# FASCICULE DE BREVET EUROPEEN

(11) **EP 1 253 927 B1**
(45) Date de publication et mention de la délivrance du brevet: **08.09.2004**
(21) Numéro de dépôt: 01904015.3
(22) Date de dépôt: 31.01.2001
(51) Int. Cl.: A61K 35/78

(54) **PROCEDE DE PREPARATION D'EXTRAITS VEGETAUX, EXTRAITS AINSI OBTENUS ET LEURS UTILISATIONS**
VERFAHREN ZUR HERSTELLUNG VON PFLANZLICHEN EXTRAKTEN, DAMIT ERHALTENE EXTRAKTEN, UND IHRE VERWENDUNGEN
METHOD FOR PREPARING PLANT EXTRACTS, PLANT EXTRACTS OBTAINED BY SAID METHOD AND USES THEREOF

(30) Priorité: 07.02.2000 FR 0001503
(43) Date de publication de la demande: 06.11.2002
(73) Titulaire: INSTITUT DES SUBSTANCES VEGETALES, 63270 Vic-le-Comte (FR)
(72) Inventeur: JEAN, Daniel, F-63270 Vic-le-Comte (FR)
(74) Mandataire: Goulard, Sophie
(86) Numéro de dépôt international: PCT/FR2001/000298
(87) Numéro de publication internationale: WO 2001/056584

(56) Documents cités:
- EP-A- 0 054 486
- DATABASE WPI Section Ch, Week 200006 Derwent Publications Ltd., London, GB; Class D16, AN 2000-065381 XP002152144 & JP 11 276148 A (WATABE Y), 12 octobre 1999 (1999-10-12)

## Description

L'Invention se rapporte à un procédé pour préparer des extraits végétaux, aux extraits végétaux susceptibles d'être obtenus par ce procédé ainsi qu'aux utilisations de ces extraits.

L'utilisation des végétaux en pharmacie, cosmétique et diététique, présente un grand intérêt car ils constituent une source extrêmement variée et étendue de substances ayant des effets bénéfiques sur la physiologie humaine et animale et, notamment, sur la santé.

A ce jour, les préparations végétales les plus fréquemment employées à des fins thérapeutiques sont obtenues par des procédés extractifs (macération, infusion, décoction, lixiviation, ...) qui ont tous en commun de comprendre une opération au cours de laquelle on fait agir un solvant (eau, alcool, éther, ...) sur la plante ou sur certaines de ses parties de manière à faire passer les principes actifs réputés être présents dans cette plante, de cette dernière vers le solvant. On distingue ainsi, selon l'état (frais ou sec) dans lequel se trouve la plante ou les parties traitées, le procédé d'extraction et le solvant extractif utilisés, les teintures mères, les teintures alcooliques, les alcoolatures et les extraits, ces derniers résultant d'une opération complémentaire de concentration destinée à augmenter leur teneur en principes actifs. Toutes ces préparations ont pour caractéristique de ne renfermer que les substances initialement présentes dans la plante, qui sont solubles dans le solvant utilisé lors de l'opération d'extraction.

Si un grand nombre des substances actives présentes dans le règne végétal sont maintenant bien connues, il existe néanmoins de nombreuses espèces végétales dont on sait qu'elles ont des vertus thérapeutiques, mais dont on ignore encore quelles sont les substances qu'elles renferment et qui sont à l'origine de ces vertus.

De plus, il s'avère que, dans un certain nombre de cas, des substances réputées être responsables de l'effet thérapeutique d'une plante, n'apportent pas, lorsqu'elles sont utilisées de manière isolée, le même bénéfice thérapeutique que celui procuré par l'ensemble de la plante. Ainsi, cet effet thérapeutique pourrait être lié en partie à des phénomènes de synergies ou d'interactions positives entre les différents constituants des plantes et, notamment, entre les substances qui ont été identifiées et celles qui ne l'ont pas été.

Enfin, bon nombre de remèdes traditionnels à base de plantes se réfèrent à des pratiques séculaires où la notion de principe actif était encore inconnue, et il existe tout un corpus décrivant l'usage de plantes dans leur totalité à des fins médicinales.

De ce fait, les professionnels de la phytothérapie souhaitent disposer, de préférence, de préparations végétales dites "*totales*", c'est-à-dire renfermant, pour une plante déterminée, tous les composants actifs, connus et inconnus, de cette plante, plutôt que de principes actifs bien identifiés et se présentant sous une forme purifiée ou partiellement purifiée.

C'est la raison pour laquelle, depuis quelques années, bon nombre de produits de phytothérapie sont proposés sous la forme de poudres obtenues par broyage de plantes sèches ou par lyophilisation de plantes fraîches, et qui sont mises directement en gélules ou en comprimés lorsqu'elles sont destinées à être utilisées comme médicaments, produits cosmétiques ou compléments alimentaires par les consommateurs. On connaît également des suspensions dites "*intégrales*" qui sont obtenues en traitant des plantes fraîches par le froid de manière à bloquer les réactions enzymatiques. Cependant, toutes ces préparations présentent l'inconvénient majeur de renfermer des composés comme la cellulose, les gommes, les mucilages, ou des tanins condensés, qui, non seulement peuvent être dénués de tout intérêt thérapeutique, mais réduisent, par leur présence, la biodisponibilité des principes actifs. De plus, on sait qu'au cours du séchage d'une plante, un certain nombre des composés qu'elle renferme se dégradent, comme les enzymes et les vitamines. Aussi, les poudres préparées à partir de plantes sèches sont-elles inaptes à restituer l'intégralité des substances actives initialement présentes dans ces plantes.

La réalisation de préparations végétales "*totales*" par reconstitution, c'est-à-dire par réunion des différentes substances actives censées être présentes dans une plante déterminée, ne peut être envisagée, dans la mesure où, si le contenu biochimique des plantes est susceptible d'être en grande partie déterminé par l'analyse, il s'avère que certains composants des végétaux ne peuvent être détectés même par des techniques d'analyse très performantes comme les chromatographies en phase liquide ou en phase gazeuse couplées à la spectrométrie de masse. Tel est le cas notamment des substances qui figurent à l'état de traces et de celles qui relèvent de structures très complexes comme les polyphénols, les protéines et les polysaccharides.

L'Inventeur s'est, donc, fixé pour but de fournir un procédé qui permette, d'une façon générale, d'obtenir des préparations dont la composition soit aussi proche que possible du contenu biochimique des végétaux à partir desquels elles sont élaborées, tout en étant débarrassées des composants dénués d'intérêt du type cellulose, gommes, mucilages et autres.

L'Inventeur s'est, plus particulièrement, fixé pour but de fournir un procédé qui garantisse la présence, dans ces préparations, de tous les composants qui participent aux fonctions vitales des végétaux, à savoir le catabolisme cellulaire pour tous les organes et la photosynthèse pour les organes chlorophylliens, ainsi que la majeure partie des composants issus du "*métabolisme secondaire*", et qui représentent le plus souvent les substances actives présentant un intérêt pour les phytothérapeutes.

L'Inventeur s'est, encore, fixé pour but de fournir un procédé d'extraction qui garantisse également que tous les composants présents dans lesdites préparations, y compris les plus fragiles, ont conservé leur intégrité biochimique et, partant, toute leur aptitude à être actifs.

L'Inventeur s'est, en outre, fixé pour but de fournir un procédé qui, tout en présentant les avantages mentionnés ci-avant, ait un coût de mise en oeuvre compatible avec les impératifs industriels.

Ces buts sont atteints, selon l'Invention, par un procédé de préparation d'un extrait végétal, qui est caractérisé en ce qu'il comprend :
- la congélation de la plante entière ou des parties actives de cette plante à une température inférieure ou égale à -10°C,
- le broyage de cette plante ou de ses parties actives à une température de cette plante ou de ses parties actives inférieure ou égale à -20°C,
- au moins une opération d'expression du suc du broyat, cette expression étant réalisée à une température du broyat comprise entre environ -5 et 0°C.
- au moins une lixiviation du résidu de cette expression par un solvant organique miscible à l'eau, cette lixiviation étant réalisée à une température de ce résidu comprise entre environ 0 et 25°C, et
- la réunion en un même extrait du suc et des solutions extractives issue de la lixiviation, après une éventuelle concentration desdites solutions extractives.

Dans ce qui précède et dans ce qui suit, on entend par "*parties actives*" d'une plante, les parties de cette plante réputées être les plus riches en principes actifs et qui sont, par voie de conséquence, préférentiellement utilisées pour la préparation d'extraits. Selon les espèces végétales, ces parties peuvent être aériennes (tiges, feuilles, fleurs et sommités fleuries, écorces, graines, cônes, fruits, bois, ...) ou souterraines (racines, rhizomes, tubercules, ...).

Conformément à l'Invention, la congélation de la plante ou de ses parties actives est réalisée dans un délai le plus court possible à partir de leur récolte, de préférence d'au plus 12 heures, de manière à limiter les risques de dessèchement ou de fermentation de cette plante ou de ses parties actives et, partant, de dégradation de leurs composants.

Pour les mêmes raisons, lorsque la congélation de la plante ou de ses parties actives ne peut être effectuée sur le lieu de récolte, celles-ci sont avantageusement transportées jusqu'au lieu de congélation dans une enceinte dont la température interne est inférieure ou égale à 15°C et est, de préférence, d'environ 4°C, comme un camion frigorifique équipé en réfrigération positive.

Selon une première disposition préférée du procédé conforme à l'Invention, la congélation de la plante ou de ses parties actives est réalisée par leur immersion dans de l'azote liquide, auquel cas la plante ou ses parties actives sont plongées dans l'azote liquide et y sont maintenues jusqu'à ce que les transferts thermiques entre elles et ce dernier aient cessé, ce que l'on détecte par la stabilisation de l'ébullition de l'azote liquide.

Toutefois, il est également possible d'effectuer cette congélation par d'autres voies, comme par exemple en aspergeant la plante ou ses parties actives par de l'azote liquide ou en les mettant simplement au congélateur.

Une fois congelées. la plante ou ses parties actives sont soumises, soit de manière immédiate, soit de manière différée, à un broyage qui est réalisé alors que la plante ou ses parties actives présentent une température inférieure ou égale à -20°C, par exemple dans un broyeur à marteaux ou un broyeur à couteaux lui-même refroidi par une admission d'azote liquide au niveau du cylindre de broyage, de manière à écarter tout risque de décongélation des matières broyées en dépit des phénomènes d'échauffement inhérents au broyage. La température présentée par la plante ou ses parties actives au moment du broyage conditionne leur rigidité et, donc, la facilité avec laquelle elles sont susceptibles d'être broyées, et elle est avantageusement choisie de façon à ce que le broyage s'effectue avec une rapidité comparable à celle avec laquelle il se ferait s'il était réalisé sur cette même plante ou ses parties actives à l'état sec. Ainsi, d'une manière générale, les plantes très ligneuses comme l'aubier de tilleul et la bruyère, peuvent aisément être broyées à des températures comprises entre -20 et -50°C, alors que les plantes riches en résines et/ou en matières grasses comme les feuilles d'olivier et les avocats, nécessitent plutôt d'utiliser des températures inférieures à -100°C. Quant aux plantes qui ne sont ni spécialement ligneuses, ni particulièrement riches en résines et/ou en matières grasses comme la passiflore et le millepertuis, on effectue généralement leur broyage dans la gamme de températures comprises entre -50 et -100°C.

Par ailleurs, dans la mesure où le broyat est destiné à être soumis ultérieurement à une ou plusieurs lixiviations, le broyage est, de préférence, conduit jusqu'à l'obtention d'une poudre de granulométrie comprise entre environ 0,1 et 2 mm. En effet, un broyat trop grossier est difficile à épuiser par lixiviation car les solvants s'écoulent trop rapidement et ne parviennent pas à atteindre les cellules situées au coeur des éléments broyés, tandis qu'une poudre trop fine ne laisse dans sa masse que des interstices excessivement ténus dans lesquels les solvants sont retenus, empêchant l'écoulement des solutions formées et leur remplacement par du liquide extractif neuf.

Selon une autre disposition préférée du procédé conforme à l'Invention, celui-ci comprend de plus, entre le broyage de la plante ou de ses parties actives et l'opération d'expression du suc du broyat, une opération de mesure de la teneur en eau de ce broyat, qui est effectuée sur un échantillon de celui-ci pendant que le reste du broyat est maintenu en congélation.

Puis, le procédé comprend, si nécessaire, l'addition au broyat d'une quantité d'eau suffisante pour obtenir un mélange présentant une teneur en eau comprise entre environ 85 et 95%, l'Inventeur ayant, en effet, constaté que l'expression du suc du broyat s'effectue d'autant mieux que ce broyat présente une telle teneur en eau. Cette dernière peut être déterminée par l'une quelconque des méthodes classiquement employées pour mesurer le taux d'humidité d'une préparation comme celle qui consiste à apprécier la différence de poids que présente la préparation avant et après dessiccation, ou la méthode de KARL-FISCHER.

Conformément à l'Invention, l'opération d'expression du suc du broyat est réalisée alors que ce broyat se trouve à la limite de la décongélation, c'est-à-dire qu'il présente en pratique une température comprise entre -5 et 0°C. De ce fait, dès lors que la température de ce broyat est, au moment où l'on décide d'en exprimer le suc, inférieure à celle choisie pour procéder à cette expression, l'Invention prévoit de réchauffer préalablement ce dernier, de préférence en le plaçant et en le laissant à la température ambiante (environ 20-25°C), de façon à éviter qu'il ne subisse un choc thermique, et ce, jusqu'à ce que sa température atteigne la valeur désirée.

Selon encore une autre disposition préférée du procédé conforme à l'Invention, l'opération d'expression du suc du broyat est réalisée par pressage de celui-ci, par exemple avec une presse à vis (ou presse de COLAS) ou une presse hydraulique. En variante, il est possible d'utiliser une toile dans laquelle on enferme le broyat et que l'on soumet à une torsion prolongée. Par ailleurs, cette opération peut être renouvelée si désiré.

Le suc ainsi obtenu contient la majeure partie des enzymes de la plante et, d'une manière générale, l'essentiel des composants nécessaires au "*métabolisme primaire*". Aussi, renferme-t-il des substances en solution, mais également des éléments cellulaires en suspension, utiles à ce métabolisme, comme les chloroplastes, les mitochondries et autres organites cellulaires.

Selon encore une autre disposition préférée du procédé conforme à l'Invention, ce suc est ensuite débarrassé de tous les éléments qu'il renferme et dont la taille est égale ou supérieure à 200 µm, voire à 100 µm, avantageusement par une filtration à froid, par exemple par passage sur un tamis aux mailles de taille comprise entre 100 et 200 µm. On élimine ainsi les débris végétaux les plus volumineux et, partant, la plupart des constituants comme la cellulose, les gommes et les mucilages, susceptibles de freiner la biodisponibilité des substances actives, tout en conservant tous les organites cellulaires. Puis, le suc est avantageusement conservé à une température inférieure ou égale à -18°C.

Conformément à l'Invention, ce qui reste du broyat, après expression de son suc, est soumis à au moins une lixiviation (encore connue sous le nom de percolation) par un solvant organique miscible à l'eau, apte à extraire les composants issus du "*métabolisme secondaire*" de la plante (hétérosides, polyphénols, alcaloïdes, triterpènes, stérols, ...), cette lixiviation étant conduite à une température du résidu de l'expression du suc du broyat comprise entre 0 et 25°C. A titre d'exemples de solvant organique susceptible d'être utilisé au cours de cette lixiviation, on peut citer l'éthanol, le méthanol, l'acétone, l'acétonitrile, le tétrahydrofurane et leurs mélanges.

Selon un premier mode préféré de mise en oeuvre du procédé conforme à l'Invention, le résidu de l'expression du suc du broyat est soumis à une lixiviation par un solvant organique, avantageusement de l'éthanol, dont on augmente progressivement le titre au cours de cette lixiviation, de préférence, de manière régulière, par exemple par application d'un gradient linéaire. Avantageusement, cette lixiviation est initiée avec de l'eau, puis poursuivie avec le solvant organique jusqu'à ce que ce dernier atteigne un titre de 100%.

Selon un autre mode de mise en oeuvre préféré du procédé conforme à l'Invention, le résidu de l'expression du suc du broyat est soumis à plusieurs lixiviations successives qui sont toutes réalisées avec le même solvant organique, avantageusement de l'éthanol, mais en utilisant ce solvant à des titres croissant d'une lixiviation à l'autre.

En variante, le résidu de l'expression du suc du broyat peut également être soumis à plusieurs lixiviations successives qui sont réalisées avec des solvants organiques différents d'une lixiviation à l'autre, de titres identiques ou non.

Conformément à l'Invention, ces lixiviations peuvent être effectuées soit en maintenant le résidu de l'expression du suc du broyat à une température constante, soit en augmentant progressivement la température de ce résidu - ce qui a pour effet d'accélérer la vitesse d'extraction des substances qu'il renferme - et, notamment, en la faisant croître de 0 à 25°C, pour autant bien entendu de ne pas dépasser cette dernière valeur afin d'éviter une éventuelle dégradation de ces substances.

Afin d'éviter que les composants actifs de la plante ou de ses parties actives présentes dans l'extrait, une fois celui-ci préparé, ne se dégradent ultérieurement, notamment au cours des phases de stockage et de transport de cet extrait, on préfère, dans le cadre de la présente Invention, préparer ledit extrait de façon à ce qu'il se présente soit sous une forme sèche, soit en solution dans un solvant organique non volatile, miscible à l'eau et non toxique, anhydre ou éventuellement en association avec une faible quantité d'eau, c'est-à-dire au plus égale à 15 %.

Dans le cas où l'on souhaite préparer un extrait sec, l'Invention prévoit de procéder, de préférence, à une concentration des solutions extractives issues de la ou des lixiviations, de manière à en éliminer le ou les solvants organiques et à en réduire le volume d'eau, avant de réunir ces solutions extractives et le suc résultant de l'expression du broyat.

Dans ce cas, les solutions extractives, qui peuvent être aqueuses, hydro-organiques ou purement organiques, sont avantageusement réunies, puis concentrées sous vide, à pression réduite et à une température d'au plus 35°C, jusqu'à l'obtention d'une solution aqueuse résiduelle dont le volume est choisi en fonction de la concentration finale en matières sèches compatible avec une lyophilisation, dans de bonnes conditions, c'est-à-dire inférieure ou égale à 25 % de matières sèches.

En pratique, ce volume est déterminé à partir des teneurs en matières sèches des solutions extractives et du suc résultant de l'expression du broyat, que l'on mesure préalablement sur des échantillons de ceux-ci, par exemple en les soumettant à une dessiccation.

Cette solution aqueuse résiduelle est ajoutée au suc résultant de l'expression du broyat, après une éventuelle décongélation de ce dernier, et l'ensemble est séché, de préférence par lyophilisation, et homogénéisé.

La poudre ainsi obtenue peut être conditionnée telle quelle, par exemple en gélules, en sachets ou en comprimés ou encore être mise en suspension dans un liquide non aqueux comme une huile végétale ou un solvant gras physiologiquement acceptable en vue de son utilisation par les consommateurs en tant que médicament, produit cosmétique ou complément alimentaire. Elle peut également servir de matière première pour la préparation de formulations plus complexes à visée médicamenteuse, cosmétique ou nutritionnelle, et se présentant sous la forme de gélules, de granules, de comprimés ou de tablettes.

Dans le cas où l'on souhaite préparer un extrait en solution dans un solvant organique non volatile, miscible à l'eau et non toxique, l'Invention prévoit d'ajouter ce solvant, qui est avantageusement choisi parmi le glycérol, le monopropylène glycol et les sirops de glucose et de fructose, aux solutions extractives issues de la ou des lixiviations. Le volume de solvant organique non volatile ajouté à ces solutions extractives est choisi en fonction de la concentration finale en composants actifs que l'on souhaite donner à l'extrait (puisque, ce solvant n'étant pas volatile, il ne sera pas éliminé au cours des opérations de concentration) et il est, là également, déterminé à partir des teneurs en matières sèches présentées par les solutions extractives et par le suc résultant de l'expression du broyat.

Puis, les solutions extractives et le suc résultant de l'expression du broyat sont réunis, après une éventuelle décongélation de ce dernier, et l'ensemble est concentré de manière à éliminer les solvants organiques autres que ledit solvant non volatile, et l'eau ou à réduire le volume d'eau jusqu'à obtention du volume aqueux que l'on souhaite laisser dans l'extrait. Là également, ces opérations de concentration sont effectuées sous vide, à pression réduite et à une température d'au plus 35°C.

Après une éventuelle filtration, l'extrait liquide ainsi obtenu peut être conditionné, par exemple en ampoules, en flacons ou en capsules en vue de son utilisation par les consommateurs en tant que médicament, produit cosmétique ou complément alimentaire. Il peut également servir de matière première pour la préparation de formulations plus complexes à visée médicamenteuse, cosmétique ou nutritionnelle, et se présentant sous forme de solutions, de suspensions, d'émulsions ou de micro-émulsions.

Lorsque la plante ou ses parties actives sont riches en produits lipophiles du type terpènes, acides gras, lipides, huiles essentielles ou cires, le résidu de l'expression du suc du broyat peut être soumis, à l'issue de la ou des lixiviations par le(s) solvant(s) organique(s) miscible(s) à l'eau, à au moins une lixiviation supplémentaire qui est conduite au moyen d'un solvant organique non miscible à l'eau mais miscible au solvant organique utilisé au cours de la lixiviation précédente ou, si plusieurs lixiviations ont été réalisées, au solvant organique utilisé au cours de la dernière lixiviation, la température de ce résidu étant comprise entre 0 et 25°C. A titre d'exemples de solvant organique susceptible d'être utilisé au cours de cette lixiviation supplémentaire, on peut citer le butanol, l'éther, l'acétate d'éthyle, le dichlorométhane, l'hexane et leurs mélanges.

En variante, ce qui reste du broyat à l'issue de la ou des lixiviations par le(s) solvant(s) organique(s) miscible(s) à l'eau, peut être soumis à au moins une macération dans un solvant organique non miscible à l'eau du type butanol, éther, acétate d'éthyle, dichlorométhane, hexane et leurs mélanges. Cette macération est, de préférence, réalisée après avoir soumis le résidu de ladite ou desdites lixiviations à une opération d'expression, avantageusement par pressage, permettant de récupérer le(s) solvant(s) organique(s) (et les substances qu'ils contiennent) resté(s) dans ce résidu.

Dans tous les cas, les solutions extractives obtenues (lixiviats ou macérés) sont soumises à une évaporation, à pression atmosphérique et à une température de l'ordre de 45°C, qui conduit à l'obtention d'un résidu sec, dans le cas des lixiviats, ou d'un extrait pâteux, dans le cas des macérés, que l'on peut alors mélanger à l'extrait sec ou liquide issu de la ou des lixiviations conduites avec le(s) solvant(s) organique(s) miscible(s) à l'eau, pour obtenir un extrait "*total*".

Selon encore une autre disposition préférée du procédé conforme à l'Invention, celui-ci prévoit, en outre, de soumettre un échantillon de l'extrait obtenu à au moins un test de fonctionnement métabolique permettant de vérifier que l'intégrité biochimique des composants actifs de la plante ou de ses parties actives a bien été préservée au cours de leur extraction.

Avantageusement ce test de fonctionnement métabolique comprend la mesure de l'activité catabolique respiratoire intrinsèque de l'extrait. Dans le cas d'un extrait préparé à partir d'une plante chlorophyllienne, il peut être complété par une mesure de l'activité métabolique photosynthétique intrinsèque dudit extrait.

L'activité catabolique respiratoire intrinsèque d'un extrait peut être mesurée en déterminant le temps (t₁) que met un échantillon de cet extrait, placé dans une eau distillée saturée en oxygène, à abaisser de 50% le taux d'oxygène de cette eau et le temps (t₀) que met spontanément cette même eau, dans les mêmes conditions, pour perdre 50% de sa teneur en oxygène, et en calculant le rapport entre (t₁ - t₀) et le poids (P) de l'échantillon d'extrait testé.

Quant à l'activité métabolique photosynthétique intrinsèque de l'extrait, elle peut être mesurée en déterminant le temps (tₚ) que met un échantillon de l'extrait, placé dans une eau distillée présentant une teneur en oxygène de 30%, à faire monter, en présence de lumière, la teneur en oxygène de cette eau jusqu'à une valeur de 50%, et en calculant le rapport entre (tₚ) et le poids (P) de l'échantillon d'extrait testé.

Ce test de fonctionnement métabolique peut également être utilisé ultérieurement pour évaluer l'état de conservation de l'extrait au cours du temps et permettre ainsi de définir, pour cet extrait, un délai de péremption.

L'Invention a, également, pour objet un extrait végétal qui est caractérisé en ce qu'il est susceptible d'être obtenu par un procédé tel que précédemment défini.

Conformément à l'Invention, un tel extrait présente une activité métabolique détectable.

L'Invention a, aussi, pour objet cet extrait végétal comme médicament, produit cosmétique ou complément alimentaire.

L'Invention a, encore, pour objet l'utilisation d'un tel extrait pour la préparation d'un médicament, d'une composition cosmétique ou d'un complément alimentaire.

L'Invention sera mieux comprise à l'aide du complément de description qui suit et qui se réfère à des exemples de mise en oeuvre du procédé de préparation d'un extrait végétal conforme à l'Invention, de mise en oeuvre du test de fonctionnement métabolique sur des extraits ainsi préparés et de formulations élaborées à partir de ces extraits. Il va de soi, toutefois, que ces exemples sont donnés uniquement à titre d'illustrations de l'Invention et n'en constituent en aucune manière une limitation.

### EXEMPLE 1 : Préparation d'un extrait de parties aériennes de Passiflora incarnata

10 kg de parties aériennes fleuries de passiflore (*Passiflora incarnata*) sont récoltés puis transportés, à une température de 4°C, jusqu'à l'unité de préparation de l'extrait dans un délai de 12 heures où ils sont aussitôt congelés par immersion dans de l'azote liquide.

Puis, ces parties aériennes sont immédiatement soumises à un broyage, à une température de -70°C et dans un broyeur à marteaux lui-même refroidi par une admission d'azote liquide au niveau du cylindre de broyage, jusqu'à l'obtention d'une poudre de granulométrie comprise entre 1 et 2 mm.

On détermine leur teneur en eau sur un échantillon de 10 g, pendant que la poudre résultant du broyage est maintenue en congélation à -18°C. Cette teneur est de 82%. On ajoute 3 litres d'eau distillée à la poudre congelée pour obtenir une teneur en eau de 85%.

On laisse l'ensemble se réchauffer spontanément à la température ambiante jusqu'à ce qu'il présente une température de -5°C; puis on le soumet à un pressage dans une presse à vis afin d'en exprimer le suc. On obtient 6,2 litres de suc concentré à 8,4% de matières sèches, ce qui représente un poids total de matières sèches de 521 g.

Le suc obtenu par pressage est filtré sur un tamis de 100 um. Il est ensuite congelé rapidement sur des plateaux à -40°C et conservé à -18°C.

Le résidu du pressage est soumis à une lixiviation qui est conduite en augmentant progressivement la température de ce résidu de 0 à 25°C et en utilisant un gradient linéaire d'éthanol de titre croissant de 0% à 100%, obtenu par deux pompes doseuses distribuant l'eau et l'éthanol dans un réservoir tampon de 1 litre placé en amont de la cuve de lyxiviation.

Pour ce faire, le résidu du pressage est placé dans un panier en tamis d'acier inoxydable de 200 µm, qui est lui-même situé dans une cuve en acier inoxydable de 50 litres de capacité, à vidange centrale par le fond et thermostatée par une double enveloppe munie d'un système de chauffage. Cette cuve est soumise à une agitation constante. On immerge le résidu du broyat dans 50 litres d'eau distillée à une température de 1°C, puis on alimente la cuve à niveau constant régulé par une sonde de niveau reliée à une électrovanne placée sur la vidange de fond de cuve, à raison de 1 litre/heure par le gradient linéaire eau/éthanol. Cette opération est réglée de façon à ce que ce gradient atteigne 100 % d'éthanol en 48 heures. On délivre ainsi 48 litres de solvants pendant cette durée. Par ailleurs, le système de chauffage de la double enveloppe de la cuve est régulé de manière à ce que la température interne de la cuve augmente progressivement de 0 à 25°C pendant cette même durée.

Les solutions extractives recueillies sont placées en continu à -18°C. Puis, après décongélation, elles sont réunies et concentrées, sous pression réduite (environ 45 mbars) et à une température de 30°C, jusqu'à l'obtention d'une solution aqueuse résiduelle de 5 litres.

Le suc est décongelé à son tour, puis ajouté à cette solution aqueuse et résiduelle et l'ensemble est lyophilisé. On obtient ainsi 853 g d'une poudre contenant 4,4 % d'eau.

### EXEMPLE 2 : Test de fonctionnement métabolique d'un extrait de parties aériennes de Passiflora incarnata

On soumet l'extrait de parties aériennes de passiflore obtenu conformément à l'exemple 1 à un test de fonctionnement métabolique.

Pour ce faire, on place, dans une cellule cylindrique transparente à double paroi, d'une hauteur de 5 cm et d'un diamètre de 3 cm (volume total : environ 35 ml), 20 ml d'eau distillée dont la température est maintenue à 30°C par circulation d'un fluide thermostatique dans la double paroi du récipient, et qui est agitée en permanence par un barreau aimanté sans création de turbulences, ni de vortex.

On mesure le taux d'oxygène dissous dans cette eau par une sonde galvanique de mesure de l'oxygène dissous, qui est reliée à un appareil de mesure adapté, et la valeur de ce taux est enregistré.

On fait barboter de l'air dans l'eau à un débit de 50 ml/min jusqu'à ce que le taux d'oxygène dissous atteigne une valeur maximale stable correspondant à la saturation dans les conditions expérimentales. Après stabilisation, on arrête l'arrivée d'air et on enregistre la baisse progressive du taux d'oxygène dissous. On détermine le temps nécessaire à la baisse de 50 % de ce taux. Le temps t₀, qui représente le temps témoin, est de 3,65 minutes.

On fait à nouveau barboter l'air jusqu'à ce que le taux de saturation initial soit atteint. On arrête l'admission d'air. On ajoute alors 1 g d'extrait de passiflore lyophilisé et on enregistre la baisse progressive du taux d'oxygène dissous.

On détermine le temps tₗ nécessaire à la baisse de 50% de ce taux. Sa valeur est de 1,05 minutes.

La différence tᵣ entre tₗ et t₀ est égale à 2.60 minutes.

On définit l'activité catabolique respiratoire intrinsèque de l'extrait par le rapport : tᵣ/P qui est égal à 2,60 min/g.

On laisse le taux d'oxygène dissous s'abaisser jusqu'à 30 % de la saturation, puis on éclaire fortement le récipient par une lampe fluorescente VHO de 15 W placée à 25 cm de la cellule et produisant un flux de lumière blanche froide de 3 000 Lux.

On enregistre alors la remontée du taux d'oxygène dissous. On note le temps tₚ nécessaire pour atteindre à nouveau 50 % de la valeur de saturation en oxygène dissous. Ce temps est de 6,80 minutes.

On définit l'activité métabolique photosynthétique intrinsèque de l'extrait par le rapport tₚ/P qui est égal à 6,80 min/g.

### EXEMPLE 3 : Préparation d'un extrait de racines de Arctium lappa

2 kg de racines de bardane (*Arctium lappa*) sont récoltés, lavés pour en éliminer les déchets et la terre, puis transportés à une température de 10°C jusqu'à l'atelier de fabrication dans un délai de 5 heures où ils sont congelés par immersion dans de l'azote liquide.

Ces racines sont broyées immédiatement, à une température de -100°C et dans un broyeur à marteaux lui-même refroidi par une admission d'azote liquide au niveau du cylindre de broyage, jusqu'à l'obtention d'une poudre de granulométrie comprise entre 0,5 et 2 mm.

On détermine ensuite leur teneur en eau sur un échantillon de 10 g, pendant que la poudre résultant du broyage est maintenue en congélation à -18°C. Cette teneur en eau est de 87 %.

On laisse la poudre se réchauffer spontanément à température ambiante jusqu'à ce qu'elle présente une température de -5°C, puis on la soumet à un pressage dans une presse à vis afin d'en exprimer le suc.

On obtient 0,85 litre de suc concentré à 12.2% de matières sèches, ce qui représente un poids total de matières sèches de 104 g.

Ce suc est filtré sur un tamis de 100 µm. Il est ensuite congelé rapidement sur des plateaux à -40°C et conservé en congélation à -18°C.

Le résidu du pressage est soumis à trois lixiviations successives qui sont conduites à une température de ce résidu de 0°C et par trois fois 5 litres d'éthanol, respectivement à 15%, 35% et 80%. Ces lixiviations sont réalisées dans un cylindre en verre de 5 litres de capacité, fermé à sa partie inférieure par un filtre en verre fritté. Les solutions extractives recueillies sont placées aussitôt à -18°C.

Ces solutions extractives sont décongelées, puis réunies et concentrées, sous pression réduite (environ 45 mbars) et à une température de 30°C, jusqu'à l'obtention d'une solution aqueuse résiduelle de 1 litre.

Le suc est décongelé à son tour, puis ajouté à cette solution aqueuse résiduelle et l'ensemble est lyophilisé. On obtient ainsi 182 g de poudre contenant 6.6 % d'eau.

### EXEMPLE 4 : Test de fonctionnement métabolique d'un extrait de racines de Arctium lappa

On soumet l'extrait de racines de bardane obtenu conformément à l'exemple 3 à un test de fonctionnement métabolique.

Ce test est conduit dans les mêmes conditions que le test décrit dans l'exemple 2, à ceci près que le poids (P) d'extrait utilisé est de 0,5 g.

Le temps t₀ est de 3,25 minutes, tandis que le temps tₗ est de 2,92 minutes.

La différence tᵣ entre tₗ et t₀ est de 0,33 minutes.

On définit l'activité catabolique respiratoire intrinsèque de l'extrait par le rapport : tᵣ/P qui est égal à 0,66 min/g.

### EXEMPLE 5 : Préparation d'un extrait de feuilles d'Olea europea

200 kg de feuilles d'olivier (*Olea europea*) sont récoltés puis transportés à une température de 4°C jusqu'à l'atelier de fabrication dans un délai de 12 heures où ils sont congelés par aspersion par de l'azote liquide.

Les feuilles sont broyées immédiatement à une température de -120°C, dans un broyeur à marteaux lui-même refroidi par une admission d'azote liquide au niveau du cylindre de broyage, jusqu'à l'obtention d'une poudre de granulométrie comprise entre 1 et 2 mm.

On détermine leur teneur en eau sur un échantillon de 10 g, pendant que la poudre résultant du broyage est maintenue en congélation à -18°C. Cette teneur est de 71 %. On ajoute alors 26 litres d'eau distillée à la poudre congelée pour obtenir une teneur en eau de 85 %.

On laisse l'ensemble se réchauffer spontanément à température ambiante jusqu'à ce qu'il présente une température de -5°C, et on presse la masse végétale dans une presse à vis afin d'en exprimer le suc.

On obtient 82 litres de suc concentré à 8,9 % de matières sèches, ce qui représente un poids total de matières sèches de 7,3 kg.

Ce suc est filtré sur un tamis de 100 µm. Il est ensuite congelé rapidement sur des plateaux à -40°C et conservé en congélation à -18°C.

Le résidu du pressage est soumis à une lixiviation qui est conduite en augmentant progressivement la température de ce résidu de 0 à 25°C et en utilisant un gradient linéaire d'éthanol de titre croissant de 0% à 100%, obtenu par deux pompes doseuses distribuant l'eau et l'éthanol dans un réservoir tampon de 1 litre placé en amont de la cuve de lyxiviation.

Pour ce faire, le résidu du pressage est placé dans un panier en tamis d'acier inoxydable de 200 µm, qui est lui-même situé dans une cuve en acier inoxydable de 350 litres de capacité, à vidange centrale par le fond et thermostatée par une double enveloppe munie d'un système de chauffage. Cette cuve est soumise à une agitation constante. On immerge le résidu du broyat dans 700 litres d'eau distillée à une température de 1°C, puis on alimente la cuve à niveau constant régulé par une sonde de niveau reliée à une électrovanne placée sur la vidange de fond de cuve, à raison de 12 litres/heure par le gradient linéaire eau/éthanol. Cette opération est réglée de façon à ce que ce gradient atteigne 100 % d'éthanol en 48 heures. On délivre ainsi 576 litres de solvants pendant cette durée. Par ailleurs, le système de chauffage de la double enveloppe de la cuve est régulé de manière à ce que la température interne de la cuve augmente progressivement de 0 à 25°C pendant cette même durée.

Les solutions extractives recueillies sont placées en continu à -18°C.

A l'issue de cette lixiviation, le résidu de cette lixiviation est sorti de la cuve, puis soumis à un pressage dans une presse à vis. L'éthanol restant dans ce résidu est mis à évaporer à l'air libre.

On procède ensuite à deux macérations successives de ce résidu, chacune dans 600 litres de chlorure de méthylène. Les solutions extractives résultant de ces macérations sont réunies puis évaporées à pression atmosphérique à 45°C. On obtient 13 kg d'un extrait pâteux.

Parallèlement, les solutions extractives issues de la lixiviation et le suc sont décongelés puis réunis. L'ensemble est concentré, sous pression réduite (environ 45 mbars) et à une température de 30°C, jusqu'à l'obtention d'une solution aqueuse résiduelle de 100 litres.

Cette dernière est lyophilisée. On obtient 18 kg d'une poudre contenant 5.3 % d'eau.

L'extrait pâteux issu des macérations dans le chlorure de méthylène est mélangé progressivement à cette poudre pour obtenir un extrait "*total*" de feuilles d'olivier.

### EXEMPLE 6 : Test de fonctionnement métabolique d'un extrait de feuilles d'Olea europea

On soumet l'extrait de feuilles d'olivier obtenu conformément à l'exemple 5 à un test de fonctionnement métabolique.

Ce test est conduit dans les mêmes conditions que le test décrit dans l'exemple 2.

Le temps t₀ est de 3,35 minutes, le temps tₗ est de 2,55 minutes, tandis que le temps tₚ est de 8,90 minutes.

L'activité catabolique respiratoire intrinsèque de l'extrait est donc égale à 0,80 min/g, tandis que son activité métabolique photosynthétique intrinsèque est égale à 8,90 min/g.

### EXEMPLE 7 : Préparation d'un extrait de parties aériennes de Hypericum perforatum

10 kg de parties aériennes fleuries de millepertuis (*Hypericum perforatum*) sont récoltés puis transportés à une température de 4°C jusqu'à l'atelier de fabrication dans un délai de 12 heures où ils sont congelés par immersion dans de l'azote liquide.

Ces parties aériennes fleuries sont broyées immédiatement, à une température de -70°C et dans un broyeur à marteaux lui-même refroidi par une admission d'azote liquide au niveau du cylindre de broyage, jusqu'à l'obtention d'une poudre de granulométrie comprise entre 1 et 3 mm.

On détermine leur teneur en eau sur un échantillon de 10 g, pendant que la poudre résultant du broyage est maintenue en congélation à -18°C. La valeur trouvée est de 88 % d'eau.

On laisse la poudre se réchauffer spontanément à température ambiante jusqu'à ce qu'elle présente une température de -5°C, et on la presse dans une presse à vis afin d'en exprimer le suc.

On obtient ainsi 5,5 litres de suc concentré à 12 % de matières sèches, soit un poids total de matières sèches de 660 g.

Ce suc est filtré sur un tamis de 100 µm, puis congelé rapidement sur des plateaux à -40°C et conservé en congélation à -18°C.

Le résidu du pressage est soumis à une lixiviation qui est conduite dans les mêmes conditions que celle décrite dans l'exemple 1, à ceci près que la cuve de lixiviation est alimentée par le gradient linéaire d'éthanol à raison de 1,5 litre/heure. On délivre donc 72 litres de solvants pendant les 48 heures que dure la lixiviation. Les solutions extractives recueillies sont placées en continu à -18°C.

Après décongélation, ces solutions extractives sont réunies et concentrées, sous pression réduite (environ 45 mbars) et à une température de 30°C, jusqu'à l'obtention d'une solution aqueuse résiduelle de 5 litres.

Le suc est décongelé à son tour et ajouté à cette solution aqueuse résiduelle et l'ensemble est lyophilisé. On obtient ainsi 1 350 g d'une poudre contenant 6,4 % d'eau.

### EXEMPLE 8 : Test de fonctionnement métabolique d'un extrait de parties aériennes de Hypericum perforatum

On soumet l'extrait de parties aériennes de millepertuis obtenu conformément à l'exemple 7 à un test de fonctionnement métabolique.

Ce test est conduit dans les mêmes conditions que le test décrit dans l'exemple 2.

Le temps t₀ est de 3,55 minutes, le temps tₗ est de 2,05 minutes, tandis que le temps tₚ est de 7.50 minutes.

L'activité catabolique respiratoire intrinsèque de l'extrait est donc égale à 1,50 min/g, tandis que son activité métabolique photosynthétique intrinsèque est égale à 7.50 min/g.

### EXEMPLE 9 : Préparation de gélules à partir d'un extrait de parties aériennes de Hypericum perforatum

Un lot de 100 000 gélules n° 0 est préparé par remplissage unitaire de 300 mg d'une poudre présentant la composition suivante :

| | |
|---|---|
| Extrait lyophilisé de parties aériennes de *Hypericum Perforatum* préparé conformément à l'exemple 7 | 5 000 g |
| Maltodextrine | 20 000 g |
| Silice | 5 000 g |

L'extrait, la maltodextrine et la silice sont mélangés dans un mélangeur cubique, puis le mélange résultant est utilisé pour remplir les gélules sur une machine semi-automatique à remplissage par arasement contraint.

### EXEMPLE 10: Préparation de comprimés à partir d'un extrait de parties aériennes de Passiflora incarnata

Un lot de 100 000 comprimés est préparé par granulation humide d'un mélange présentant la formule suivante :

| | |
|---|---|
| Extrait lyophilisé de *Passiflora incarnata* préparé conformément à l'exemple 1 | 10 000 g |
| Amidon | 1 000 g |
| Stéarate de magnésium | 2 500 g |
| Talc | 1 000 g |
| Silice microcristalline | 0,5 g |

Les différents composants sont mélangés dans un mélangeur cubique jusqu'à complète homogénéité, puis le mélange résultant est mouillé par 10 kg d'éthanol dénaturé à 96 %. Il est soumis à une granulation humide. Les granulés sont séchés dans un appareil à lit d'air fluidisé, puis pulvérisés.

La compression est assurée par une machine à comprimés alternative pour obtenir environ 100 000 comprimés de 150 mg chacun et contenant 50 mg d'extrait de parties aériennes de passiflore.

### EXEMPLE 11 : Préparation d'un extrait en solution anhydre de fleurs de Calendula officinalis

50 kg de fleurs de calendula (*Calendula Offlcinalis*) sont récoltés puis transportés à une température de 4°C jusqu'à l'atelier de fabrication dans un délai de 6 heures où ils sont congelés par immersion dans de l'azote liquide.

Les fleurs sont broyées immédiatement, à une température de -90°C dans un broyeur à marteaux lui-même refroidi par une admission d'azote liquide au niveau du cylindre de broyage, jusqu'à l'obtention d'une poudre de granulométrie comprise entre 0,2 et 1 mm.

On détermine leur teneur en eau sur un échantillon de 10 g, pendant que la poudre résultant du broyage est maintenue en congélation à -18°C. La valeur trouvée est de 92 % d'eau.

On laisse cette poudre se réchauffer spontanément à température ambiante jusqu'à ce qu'elle présente une température de -5°C, et on la presse dans une presse à vis afin d'en exprimer le suc.

On obtient 22 litres de suc concentré à 9 % de matières sèches, ce qui représente un poids total de matières sèches de 1 980 g.

Ce suc est filtré sur un tamis de 100µm, puis il est congelé rapidement sur des plateaux à -40°C et conservé en congélation à -18°C.

Le résidu du pressage est ensuite soumis à une lixiviation qui est conduite en augmentant progressivement la température de 0°C à 25°C et en utilisant un gradient linéaire d'éthanol de titre croissant de 0% à 100%, obtenu par deux pompes doseuses distribuant l'eau et l'éthanol dans un réservoir tampon de 1 litre placé en amont de la cuve de lyxiviation.

Cette lixiviation est réalisée dans les mêmes conditions que celle décrite dans l'exemple 1, à ceci près que la cuve de lixiviation est alimentée par le gradient linéaire d'éthanol à raison de 2 litres/heure. On délivre donc 96 litres de solvants pendant les 48 heures que dure cette lixiviation. Les solutions recueillies sont placées en continu à -18°C.

Après décongélation, les solutions extractives sont réunies et mélangées à 25 kg de glycérol. Puis, ce mélange est concentré, sous pression réduite (environ 5 mbars) et à une température de 30°C. jusqu'à la fin de la distillation de l'eau résiduelle. On obtient ainsi 29 kg d'une solution contenant 6 % d'eau.

## Revendications

1. Procédé de préparation d'un extrait végétal, **caractérisé en ce qu'**il comprend :
- la congélation de la plante entière ou des parties actives de cette plante à une température inférieure ou égale à -10°C,
- le broyage de cette plante ou de ses parties actives à une température de cette plante ou de ces parties actives inférieure ou égale à -20°C,
- au moins une opération d'expression du suc du broyat, cette expression étant réalisée à une température du broyat comprise entre environ -5 et 0°C,
- au moins une lixiviation du résidu de cette expression par un solvant organique miscible à l'eau, cette lixiviation étant réalisée à une température de ce résidu comprise entre environ 0 et 25°C, et
- la réunion en un même extrait du suc et des solutions extractives issues de la lixiviation, après une éventuelle concentration desdites solutions extractives.

2. Procédé selon la revendication 1, **caractérisé en ce que** la congélation de la plante ou de ses parties actives est réalisée dans un délai le plus court possible à partir de leur récolte, de préférence d'au plus 12 heures.

3. Procédé selon la revendication 1 ou la revendication 2, **caractérisé en ce que**, lorsque la congélation de la plante ou de ses parties actives ne peut être effectuée sur le lieu de récolte, celles-ci sont transportées jusqu'au lieu de congélation dans une enceinte dont la température interne est inférieure ou égale à 15°C et est, de préférence, d'environ 4°C.

4. Procédé selon l'une quelconque des revendications précédentes, **caractérisé en ce que** la congélation de la plante ou de ses parties actives est réalisée par leur immersion dans de l'azote liquide.

5. Procédé selon l'une quelconque des revendications précédentes, **caractérisé en ce que** le broyage de la plante ou de ses parties actives est conduit jusqu'à l'obtention d'une poudre de granulométrie comprise entre environ 0,1 et 2 mm.

6. Procédé selon l'une quelconque des revendications précédentes, **caractérisé en ce qu'**il comprend, entre le broyage de la plante ou de ses parties actives et l'opération d'expression du suc du broyat, une opération de mesure de la teneur en eau de ce broyat sur un échantillon de celui-ci, le reste du broyat étant maintenu en congélation, puis, si nécessaire, l'addition à ce broyat d'une quantité d'eau suffisante pour obtenir un mélange présentant une teneur en eau comprise entre environ 85 et 95%.

7. Procédé selon la revendication 5, **caractérisé en ce qu'**il comprend, préalablement à l'opération d'expression du suc du broyat, une opération consistant à réchauffer celui-ci, de préférence en le plaçant et en le laissant à la température ambiante, jusqu'à ce qu'il présente la température choisie pour procéder à cette expression.

8. Procédé selon l'une quelconque des revendications précédentes, **caractérisé en ce que** l'expression du suc du broyat est réalisée par pressage de celui-ci.

9. Procédé selon l'une quelconque des revendications précédentes, **caractérisé en ce que** le suc est débarrassé de tous les éléments qu'il renferme dont la taille est égale ou supérieure à 200 µm et, de préférence, à 100 µm, avantageusement par une filtration à froid.

10. Procédé selon l'une quelconque des revendications précédentes, **caractérisé en ce que** le résidu de l'expression du suc du broyat est soumis à une lixiviation par un solvant organique dont on augmente progressivement le titre au cours de cette lixiviation, de préférence de manière régulière.

11. Procédé selon la revendication 10, **caractérisé en ce que** la lixiviation est initiée avec de l'eau, puis poursuivie avec le solvant organique jusqu'à ce que ce dernier atteigne un titre de 100%.

12. Procédé selon l'une quelconque des revendications 1 à 9, **caractérisé en ce que** le résidu de l'expression du suc du broyat est soumis à plusieurs lixiviations successives qui sont toutes réalisées avec le même solvant organique, mais en utilisant ce solvant à des titres croissant d'une lixiviation à l'autre.

13. Procédé selon l'une quelconque des revendications précédentes, **caractérisé en ce que** ce que l'on augmente progressivement la température du résidu de l'expression du suc de broyat au cours de la ou desdites lixiviations.

14. Procédé selon l'une quelconque des revendications précédentes **caractérisé en ce que** le solvant organique utilisé au cours de la ou des lixiviations est choisi parmi l'éthanol, le méthanol, l'acétone, l'acétonitrile, le tétrahydrofurane et leurs mélanges.

15. Procédé selon l'une quelconque des revendications précédentes, **caractérisé en ce que**, pour préparer un extrait sec, les solutions extractives issues de la ou des lixiviations sont concentrées avant d'être réunies avec le suc résultant de l'expression du broyat.

16. Procédé selon la revendication 15, **caractérisé en ce que** la solution aqueuse résultant de la concentration desdites solutions extractives et le suc résultant de l'expression du broyat, une fois réunis, sont soumis à une déshydratation, de préférence par lyophilisation.

17. Procédé selon l'une quelconque des revendications 1 à 14, **caractérisé en ce que**, pour préparer un extrait en solution dans un solvant organique non volatile, miscible à l'eau et non toxique, et présentant une teneur en eau au plus égale à 15%, ce solvant est ajouté aux solutions extractives issues de la ou des lixiviations, puis ces solutions extractives et le suc résultant de l'expression du broyat sont réunis et l'ensemble est concentré pour éliminer le(s) solvant(s) organique(s) autres que ledit solvant non volatile, et l'eau ou pour réduire le volume d'eau jusqu'à obtention du volume aqueux que l'on souhaite laisser dans ledit extrait.

18. Procédé selon l'une quelconque des revendications 10 à 17, **caractérisé en ce que** le résidu de l'expression du suc du broyat est soumis, à l'issue de la ou des lixiviations par le(s) solvant(s) organique(s) miscible(s) à l'eau, à au moins une lixiviation supplémentaire qui est conduite au moyen d'un solvant organique non miscible à l'eau mais miscible au solvant organique utilisé au cours de la lixiviation précédente ou, si plusieurs lixiviations ont été réalisées, au solvant organique utilisé au cours de la dernière lixiviation, la température de ce résidu étant comprise entre 0 et 25°C.

19. Procédé selon l'une quelconque des revendications 10 à 17, **caractérisé en ce que** le résidu de l'expression du suc du broyat est soumis, à l'issue de la ou des lixiviations par le(s) solvant(s) organique(s) miscible(s) à l'eau, à au moins une macération dans un solvant organique non miscible à l'eau.

20. Procédé selon la revendication 18 ou la revendication 19, **caractérisé en ce que** les solutions extractives obtenues (lixiviats ou macérés) sont soumises à une évaporation, à pression atmosphérique et à une température de l'ordre de 45°C, pour obtenir un résidu sec, dans le cas des lixiviats, ou un extrait pâteux, dans le cas des macérés, qui est mélangé à l'extrait sec ou liquide issu de la ou des lixiviations conduites avec le(s) solvant(s) organique(s) miscible(s) à l'eau.

21. Procédé selon l'une quelconque des revendications précédentes, **caractérisé en ce qu'**il comprend une étape consistant à soumettre l'extrait à un test de fonctionnement métabolique.

22. Procédé selon la revendication 21, **caractérisé en ce que** le test de fonctionnement métabolique comprend la mesure de l'activité catabolique respiratoire intrinsèque de l'extrait.

23. Extrait végétal obtenu par le procédé de préparation selon l'une quelconque des revendications 1 à 20, **caractérisé en ce qu'**il consiste en la réunion, en un même extrait, du suc résultant de l'expression du broyat et des solutions extractives issues de la lixiviation et **en ce que** ledit suc comprend des organites cellulaires.

24. Extrait végétal selon la revendication 23, **caractérisé en ce qu'**il présente une activité métabolique détectable.

25. Extrait végétal selon la revendication 23 ou la revendication 24 comme médicament, produit cosmétique ou complément alimentaire.

26. Utilisation d'un extrait végétal selon la revendication 23 ou la revendication 24 pour la préparation d'un médicament, d'une composition cosmétique ou d'un complément alimentaire.

## Patentansprüche

1. Verfahren zur Herstellung eines Pflanzenextrakts, **dadurch gekennzeichnet, daß** es folgende Schritte umfaßt:
- Einfrieren der gesamten Pflanze oder der aktiven Teile dieser Pflanze bei einer Temperatur unter oder gleich -10° C,
- Zerkleinern dieser Pflanze oder ihrer aktiven Teile bei einer Temperatur der Pflanze oder von deren aktiven Teilen unter oder gleich -20° C,
- wenigstens einem Auspressen des Saftes des zerkleinerten Gutes, wobei das Auspressen bei einer Temperatur des zerkleinerten Gutes im Bereich zwischen - 5 und 0° C durchgeführt wird,
- wenigstens einem Auslaugen des Rückstands des Auspressens mit einem mit Wasser mischbaren organischen Lösungsmittel, wobei dieses Auslaugen bei einer Temperatur des Rückstands im Bereich zwischen 0 und 25° C durchgeführt wird, und
- Vereinigen Saftes und der vom Auslaugen stammenden Extraktionslösungen zu einem Extrakt nach einem eventuellen Aufkonzentrieren der genannten Extraktionslösungen.

2. Verfahren nach Anspruch 1, **dadurch gekennzeichnet, daß** das Einfrieren der Pflanze oder von deren aktiven Teilen mit geringstmöglicher Verzögerung nach der Ernte erfolgt, bevorzugt nach höchstens 12 Stunden.

3. Verfahren nach Anspruch 1 oder 2, **dadurch gekennzeichnet, daß** wenn das Einfrieren der Pflanze oder von deren aktiven Teilen nicht am Ernteort bewirkt werden kann, dieselben in einem abgeschlossenen Raum zum Ort des Einfrierens transportiert werden, dessen Innentemperatur unter oder gleich 15° C ist, bevorzugt etwa 4° C.

4. Verfahren gemäß einem der vorhergehenden Ansprüche, **dadurch gekennzeichnet, daß** das Einfrieren der Pflanze oder von deren aktiven Teilen durch Eintauchen derselben in flüssigen Stickstoff bewirkt wird.

5. Verfahren gemäß einem der vorhergehenden Ansprüche, **dadurch gekennzeichnet, daß** das Zerkleinern der Pflanze oder von deren aktiven Teilen bis zum Erhalt eines Pulvergranulats im Bereich zwischen 0,1 und 2 mm durchgeführt wird.

6. Verfahren gemäß einem der vorhergehenden Ansprüche, **dadurch gekennzeichnet, daß** es zwischen dem Zerkleinern der Pflanze oder von seinen aktiven Teilen und dem Auspressen des Saftes des zerkleinerten Gutes ein Messen des Wassergehalts des zerkleinerten Gutes anhand einer Probe desselben umfaßt, wobei der Rest des zerkleinerten Gutes beim Einfrieren gehalten wird und dann, wenn erforderlich, die Zugabe einer ausreichenden Menge Wasser zu dem zerkleinerten Gut zum Erhalt einer Mischung mit einem Wassergehalt im Bereich zwischen 85 und 95% erfolgt.

7. Verfahren gemäß Anspruch 5, **dadurch gekennzeichnet, daß** es, vor dem Auspressen des Saftes des zerkleinerten Gutes, ein Erwärmen desselben umfaßt, wobei es bevorzugt bei Umgebungstemperatur plaziert und belassen wird, bis es die zum Auspressen gewählte Temperatur aufweist.

8. Verfahren nach einem der vorhergehenden Ansprüche, **dadurch gekennzeichnet, daß** das Auspressen des Saftes des zerkleinerten Gutes durch Pressen desselben. bewirkt wird.

9. Verfahren gemäß einem der vorhergehenden Ansprüche, **dadurch gekennzeichnet**, der Saft von sämtlichen Elementen befreit wird, die er enthält und deren Größe gleich oder größer als 200 µm ist und, bevorzugt, 100 µm, vorteilhafterweise mittels einer Kaltfiltration.

10. Verfahren gemäß einem der vorhergehenden Ansprüche, **dadurch gekennzeichnet, daß** der Rückstand des Auspressens des Saftes des zerkleinerten Gutes einem Auslaugen mit einem organischen Lösungsmittel unterworfen wird, dessen Titer im Verlauf des Auslaugens progressiv ansteigt, bevorzugt auf gleichmäßige Weise.

11. Verfahren gemäß Anspruch 10, **dadurch gekennzeichnet, daß** das Auslaugen mit Wasser begonnen wird, nachfolgend mit dem organischen Lösungsmittel bis das letztere einen Titer von 100% erreicht.

12. Verfahren gemäß einem der Ansprüche 1 bis 9, **dadurch gekennzeichnet, daß** der Rückstand des Auspressens des Saftes des zerkleinerten Gutes mehreren aufeinanderfolgenden Auslaugungen unterworfen wird, die sämtlich mit dem selben organischen Lösungsmittel bewirkt werden, man aber das Lösungsmittel mit überkreuzten Titern von einem Auslaugen zum andern verwendet.

13. Verfahren gemäß einem der vorhergehenden Ansprüche, **dadurch gekennzeichnet, daß** man die Temperatur des Rückstands des Auspressens des Saftes des zerkleinerten Gutes im Verlauf der oder der genannten Auslaugungen progressiv erhöht.

14. Verfahren gemäß einem der vorhergehenden Ansprüche, **dadurch gekennzeichnet, daß** das im Verlauf der einen oder mehreren Auslaugungen verwendete organische Lösungsmittel ausgewählt ist aus Ethanol, Methanol, Aceton, Acetonitril, Tetrahydrofuran und deren Mischungen.

15. Verfahren gemäß einem der vorhergehenden Ansprüche, **dadurch gekennzeichnet, daß** die aus der einen oder den mehreren Auslaugungen stammenden Extraktionslösungen zum Herstellen eines Trockenextrakts vor der Vereinigung mit dem aus dem Auspressen des zerkleinerten Gutes resultierenden Saft aufkonzentriert werden.

16. Verfahren gemäß Anspruch 15, **dadurch gekennzeichnet, daß** die aus dem Aufkonzentrieren der genannten Extraktionslösungen und der aus dem Auspressen des zerkleinerten Gutes resultierenden Saftes resultierende wäßrige Lösung, einmal vereinigt, einer Dehydratation unterworfen werden, bevorzugt einem Gefriertrocknen.

17. Verfahren gemäß einem der Ansprüche 1 bis 14, **dadurch gekennzeichnet, daß** zur Herstellung eines Extrakts in Lösung in einem nicht-flüchtigen, mit Wasser mischbaren und nicht toxischen organischen Lösungsmittel mit einem Wassergehalt von mehr als 15% das Lösungsmittel zu den aus dem einen oder mehreren Auslaugungen stammenden Extraktionslösungen gegeben wird, wobei diese Extraktionslösungen und der aus dem Auspressen des zerkleinerten Gutes stammende Saft vereinigt werden und das Ganze zum Entfernen von dem (den) anderen organischen Lösungsmittel(n) als dem genannten nicht-flüchtigen Lösungsmittel und Wasser zum Reduzieren des Wasservolumens bis zum Erhalt eines wäßrigen Volumens, welches man im genannten Extrakt belassen wünscht, konzentriert.

18. Verfahren gemäß einem der Ansprüche 10 bis 17, **dadurch gekennzeichnet, daß** der Rückstand des Auspressens des Saftes des zerkleinerten Gutes mit dem (den) aus dem einen oder mehreren Auslaugungen stammenden, mit Wasser mischbaren organischen Lösungsmittel(n) wenigstens einem zusätzlichen Auslaugen unterworfen wird, das mit einem nicht mit Wasser, aber mit einem im Verlauf des vorhergehenden Auslaugens oder, wenn mehrere Auslaugungen durchgeführt wurden, mit dem im Verlauf des letzten Auslaugens mischbaren organischen Lösungsmittel, wobei die Temperatur des Rückstands in dem Bereich zwischen 0 und 25°C enthalten ist.

19. Verfahren gemäß einem der Ansprüche 10 bis 17, **dadurch gekennzeichnet, daß** der Rückstand des Auspressens des Saftes des zerkleinerten Gutes mit dem (den) aus der einen oder mehreren Auslaugungen stammenden, mit Wasser mischbaren Lösungsmittel(n) wenigstens einer Mazeration in einem nicht mit Wasser mischbaren organischen Lösungsmittel unterworfen wird.

20. Verfahren gemäß Anspruch 18 oder Anspruch 19, **dadurch gekennzeichnet, daß** die erhaltenen Extraktionslösungen (Auslaugungen oder Mazerate) einem Verdampfen bei Atmosphärendruck und einer Temperatur in der Größenordnung von 45°C unterworfen werden, um im Fall der Auslaugungen einen Trockenrückstand zu erhalten oder, im Fall der Mazerate einen pastösen Extrakt, der mit dem Trocken- oder Flüssigextrakt, der aus der einen oder mehreren Auslaugungen, die mit dem (den) mit Wasser mischbaren organischen Lösungsmittel(n) durchgeführt wurden, gemischt wird.

21. Verfahren gemäß einem der vorhergehenden Ansprüche, **dadurch gekennzeichnet, daß** es eine Stufe umfaßt, die aus dem Unterwerfen des Extrakts unter einen metabolischen Funktionstest besteht.

22. Verfahren gemäß Anspruch 21, **dadurch gekennzeichnet, daß** der Test der metabolischen Funktion die Messung der dem Extrakt innewohnenden katabolischen Atmungsaktivität umfaßt.

23. Pflanzenextrakt, erhalten durch ein Verfahren gemäß einem der Ansprüche 1 bis 20, **dadurch gekennzeichnet, daß** es in der Vereinigung, in einem Extrakt, des aus dem Auspressen des zerkleinerten Gutes resultierenden Saftes und der aus dem Auslaugen stammenden Extraktionslösungen besteht und daß der genannte Saft zelluläre Organite umfaßt.

24. Pflanzenextrakt nach Anspruch 23, **dadurch gekennzeichnet, daß** er eine nachweisbare metabolische Aktivität zeigt.

25. Pflanzenextrakt nach Anspruch 23 oder Anspruch 24 als Medikament, kosmetisches Produkt oder Nahrungsergänzungsmittel.

26. Verwendung eines Pflanzenextrakts nach Anspruch 13 oder Anspruch 14 zur Herstellung eines Medikaments, einer kosmetischen Zusammensetzung oder von einem Nahrungsergänzungsmittel.

## Claims

1. Process for preparing a vegetable extract, **characterized in that** it comprises:
- freezing the whole plant or the active parts of this plant at a temperature below or equal to -10°C,
- comminuting (crushing/grinding) this plant or its active parts while this plant or these active parts are at a temperature below or equal to -20°C,
- at least one operation of expressing the juice from the comminuted material, this expression being performed while the temperature of the comminuted material is comprised between about -5 and 0°C,
- at least one lixiviation of the residue from this expression process by a water-miscible organic solvent, this lixiviation being performed at a temperature of this residue comprised between about 0 and 25°C, and
- combining into a single extract the juice and the solutions of extract resulting from the lixiviation, after optional concentration of the said extract solutions.

2. Process according to Claim 1, **characterized in that** the freezing of the plant or of its active parts is carried out within the shortest possible period of time following their harvesting, preferably a maximum of 12 hours.

3. Process according to Claim I or Claim 2, **characterized in that**, when the freezing of the plant or of its active parts cannot be performed at the place where they are harvested, they are transported to the freezing facility in an enclosure whose internal temperature is below or equal to 15°C and is preferably about 4°C.

4. Process according to any of the foregoing Claims, **characterized in that** the freezing of the plant or of its active parts is performed by immersion in liquid nitrogen.

5. Process according to any of the foregoing Claims, **characterized in that** the comminuting of the plant or of its active parts is performed until a powder with a particle size comprised between about 0.1 and 2 mm is obtained.

6. Process according to any of the foregoing Claims, **characterized in that** it comprises, between the comminuting of the plant or of its active parts and the operation of expressing the juice from the comminuted material, an operation to measure the water content of this comminuted material on a sample of the latter, the remainder of the comminuted material being kept frozen, and then, if necessary, adding to this comminuted material a quantity of water sufficient to obtain a mixture having a water content comprised between about 85 and 95 %.

7. Process according to Claim 5, **characterized in that** it comprises, prior to the operation of expressing the juice from the comminuted material, an operation consisting of reheating the latter, preferably by putting it at and leaving it at ambient temperature until it has the temperature chosen to proceed to this expressing operation.

8. Process according to any of the foregoing Claims, **characterized in that** the expressing of the juice from the comminuted material is performed by pressing the latter.

9. Process according to any of the foregoing Claims, **characterized in that** the juice is freed from all the constituents contained in it whose size is equal to or greater than 200 µm, preferably 100 µm, advantageously by cold filtration.

10. Process according to any of the foregoing Claims, **characterized in that** the residue from expressing the juice from the comminuted material is subjected to lixiviation by an organic solvent whose concentration is increased progressively during this lixiviation, preferably in a regular manner.

11. Process according to Claim 10, **characterized in that** the lixiviation is begun with water, then continued with the organic solvent until the latter reaches a concentration of 100%.

12. Process according to any of the Claims 1 to 9, **characterized in that** the residue from expressing the juice from the comminuted material is subjected to several successive lixiviations, all performed with the same organic solvent, but using this solvent at concentrations that increase from one lixiviation to another.

13. Process according to any of the foregoing Claims, **characterized in that** the temperature of the residue from expressing the juice from the comminuted material is increased progressively during the said lixiviation(s).

14. Process according to any of the foregoing Claims, **characterized in that** the organic solvent used in the lixiviation(s) is chosen from ethanol, methanol, acetone, acetonitrile, tetrahydrofuran and mixtures thereof.

15. Process according to any of the foregoing Claims, **characterized in that**, in order to prepare a dry extract, the extract solutions originating from the lixiviation(s) are concentrated before combining them with the juice originating from expressing the comminuted material.

16. Process according to Claim 15, **characterized in that** the aqueous solution resulting from concentrating the said extract solutions and the juice originating from expressing the comminuted material, after being combined, are subjected to a dehydration process, preferably by lyophilisation (freeze-drying).

17. Process according to any of the Claims 1 to 14, **characterized in that**, in order to prepare an extract in solution in a non-volatile, water-miscible, non-toxic organic solvent, and having a water content not higher than 15%, this solvent is added to the extract solutions originating from the lixiviation(s), then these extract solutions and the juice resulting from expressing the comminuted material are combined and the whole is concentrated to remove the organic solvent(s) other than the said non-volatile solvent, and the water, or to reduce the volume of water until the aqueous volume that is required to be left in the said extract is obtained.

18. Process according to any of the Claims 10 to 17, **characterized in that**, following the lixiviation(s) by the water-miscible organic solvent(s), the residue from expressing the juice from the comminuted material is subjected to at least one additional lixiviation carried out using an organic solvent that is immiscible with water but is miscible with the organic solvent used during the previous lixiviation or, if several lixiviations were performed, with the organic solvent used during the last lixiviation, the temperature of this residue being between 0 and 25°C.

19. Process according to any of the Claims 10 to 17, **characterized in that**, following the lixiviation(s) by the water-miscible organic solvent(s), the residue from expressing the juice from the comminuted material is subjected to at least one maceration in a water-immiscible organic solvent.

20. Process according to Claim 18 or Claim 19, **characterized in that** the extract solutions (lixiviates or macerates) that are obtained are subjected to an evaporation process at atmospheric pressure and at a temperature of the order of 45°C to obtain a dry residue in the case of the lixiviates, or a pasty extract in the case of the macerates, that is mixed with the dry or liquid extract originating from the lixiviation(s) performed with the water-miscible organic solvent(s).

21. Process according to any of the foregoing Claims, **characterized in that** it comprises a stage consisting of subjecting the extract to a test of metabolic functioning.

22. Process according to Claim 21, **characterized in that** the test of metabolic functioning comprises measuring the intrinsic respiratory catabolic activity of the extract.

23. Vegetable extract obtained by the preparation process according to any of the Claims 1 to 20, **characterized in that** it consists of combining into a single extract the juice resulting from expressing the comminuted material and the extract solutions originating from the lixiviation, and **in that** the said juice comprises cellular organelles.

24. Vegetable extract according to Claim 23, **characterized in that** it shows a detectable metabolic activity.

25. Vegetable extract according to Claim 23 or Claim 24 as a medicine, cosmetic product or dietary supplement.

26. Use of a vegetable extract according to Claim 23 or Claim 24 to prepare a medicine, a cosmetic composition or a dietary supplement.
